# EUROPEAN PATENT SPECIFICATION

(11) **EP 2 338 451 B1**
(45) Date of publication and mention of the grant of the patent: **07.12.2022**
(21) Application number: 09180425.2
(22) Date of filing: 22.12.2009
(51) Int. Cl.: A61F 13/539, A61F 13/532, A61F 13/53, A61F 13/15, A61F 13/472

(54) **Absorbent core with pattern of adhesive**
Saugkern mit Klebstoffmuster
Noyau absorbant doté d'un motif d'adhésif

(43) Date of publication of application: 29.06.2011
(73) Proprietor: The Procter & Gamble Company, Cincinnati, OH 45202 (US)
(72) Inventor: Carlucci, Giovanni, 66100 CHIETI (IT); Tamburro, Maurizio, 66020 SAMBUCETO (CHIETI) (IT); Toro, Evelina Sara, 66100 CHIETI (IT)
(74) Representative: Parthey, Matthias

(56) References cited:
- US-A1- 2002 115 969
- US-A1- 2007 197 987
- US-A1- 2008 312 618
- US-A1- 2009 043 273
- US-B1- 6 790 798

## Description

### FIELD OF THE INVENTION

The present invention relates to an absorbent core as part of absorbent feminine hygiene products, such as sanitary napkins and the like. Further, the present invention relates to a process for making the same.

### BACKGROUND OF THE INVENTION

Absorbent articles for absorption of body fluids such as menses or blood or vaginal discharges are well known in the art, and comprise for example feminine hygiene articles such as sanitary napkins, panty liners, tampons, interlabial devices, as well as wound dressings, and the like. When considering for example sanitary napkins, these articles typically comprise a liquid-pervious topsheet as wearer-facing layer, a backsheet as garment-facing layer and an absorbent core between topsheet and backsheet. The body fluids are acquired through the topsheet and subsequently stored in the absorbent core. The backsheet typically prevents the absorbed fluids from wetting the wearer's garment.

An absorbent core may comprise one or more fibrous absorbent materials, which in turn can comprise natural fibres, such as for example cellulose fibres, typically wood pulp fibres, synthetic fibres, or combinations thereof.

The absorbent core may also comprise super absorbent polymer materials, usually in finely dispersed form, e.g. typically in particulate form, in order to improve their absorption and retention characteristics. Super absorbent polymer materials for use in absorbent articles typically comprise water-insoluble, water-swellable, hydrogel-forming crosslinked absorbent polymers which are capable of absorbing large quantities of liquids and of retaining such absorbed liquids under moderate pressure. Super absorbent polymer materials, also known as absorbent gelling materials (AGM) can be incorporated in absorbent articles, typically in the core structure, in different ways; for example, absorbent gelling materials in particulate form can be dispersed among the fibres of fibrous layers comprised in the core, or rather localized in a more concentrated arrangement between fibrous layers.

Absorbent cores for absorbent articles having a thin structure may further provide an improved immobilization of absorbent gelling materials, particularly when the article is fully or partially loaded with liquid, and an increased wearing comfort. Such thinner structures provide absorbent articles combining better comfort, discreetness and adaptability. Such absorbent cores may for example comprise a rather low amount of fibrous material or may be free of fibrous material.

In such cores, the super absorbent polymer material typically needs to be immobilized. This can for example be done by using a thermoplastic adhesive which can be typically applied in the form of microfibers in order to enlace the super absorbent polymer material, as typically disclosed with reference to diapers as for example in patent application EP 1 447 067.

In contrast to diapers, feminine hygiene articles are intended to absorb menses instead of, or in addition to urine. As menses differ from urine, for example in terms of surface tension or flowability, it is desirable to further improve absorbent cores intended for female hygiene articles with regard to their acquisition and / or absorption properties. It may also be desirable to improve the integrity of absorbent cores.

US 2008/0312618 A1 discloses a disposable absorbent article with a sealed absorbent core. US 2009/0043273 A1 discloses an absorbent core for disposable absorbent articles, particularly for the absorption of menses or blood. US 2007/0197987 A1 discloses a method of making an absorbent composite.

### SUMMARY OF THE INVENTION

The present invention relates to an absorbent feminine hygiene product according to claim 1.

The present invention further relates to a process for making the absorbent core for the feminine hygiene product, according to claim 15.

### BRIEF DESCRIPTION OF THE DRAWINGS

Figure 1 shows a top view of an embodiment of an absorbent article.
Figure 2 shows a cross sectional view of an embodiment of an absorbent core described herein.

### DETAILED DESCRIPTION OF THE INVENTION

### Definition of terms

"Absorbent article" herein refers to an article comprising an absorbent core as will be described below. Generally, absorbent articles are capable of absorbing and storing exudates discharged from the body.

The term absorbent article generally refers to an article placed against or in proximity to the body of a wearer to absorb and contain the exudates discharged from the body.

Typical absorbent articles may be sanitary napkins, panty liners, tampons, interlabial devices, wound dressings, adult incontinence articles, diapers and the like, which are intended for the absorption of body fluids, such as menses or blood or vaginal discharges or urine.

Typical absorbent articles according to the present invention are feminine hygiene products such as sanitary napkins, panty liners, and interlabial devices.

Typically, the absorbent articles are disposable.

The term "disposable" is used herein to describe articles, which are not intended to be laundered or otherwise restored or reused as an article (i.e. they are intended to be discarded after a single use and possibly to be recycled, composted or otherwise disposed of in an environmentally compatible manner).

"Absorbent core" refers to a member of an absorbent article that is intended to absorb and store exudates, such as menses, discharged from the body. The absorbent core according to the present invention comprises at least the absorbent material, a cover layer, a substrate layer, a thermoplastic adhesive and an auxiliary adhesive.

"Absorbent material" refers to liquid absorbent materials, such as super absorbent polymer materials, fibers, foams and mixtures thereof, for example mixtures of super absorbent polymer materials with fibers. The super absorbent polymer material may herein also be referred to as AGM (absorbent gelling material). Typically, the AGM is provided in the form of super absorbent polymer particles which can be also referred to herein as "AGM particles".

"Nonwoven fabric" refers to a manufactured web of directionally or randomly orientated fibers, bonded by friction, and/or cohesion and/or adhesion, excluding paper and products which are woven, knitted, tufted, stitch-bonded incorporating binding yarns or filaments, or felted by wet-milling, whether or not additionally needled. The fibers may be of natural or man-made origin. They may be staple or continuous filaments or be formed in situ. The terms "nonwoven fabric" and "nonwoven web" are used interchangeably. The basis weight of nonwoven fabrics is usually expressed in grams per square meter (g/m²) and can be determined according to EDANA method 40.3-90. Generally, nonwoven fabrics may comprise fibers made by nature (natural fibers), made by man (synthetic fibers), or combinations thereof. Example natural fibers include but are not limited to: animal fibers such as wool, silk, fur, and hair; vegetable fibers such as cellulose, cotton, flax, linen, and hemp; and certain naturally occurring mineral fibers.

"Thermoplastic adhesive" as used herein refers to an adhesive used in the absorbent core to immobilize the absorbent material. Typically, the thermoplastic adhesive can be applied in form of microfibers to enlace the absorbent material. The thermoplastic, typically microfibrous adhesive may be a thermoplastic hot melt adhesive. The term hot melt adhesive refers to an adhesive applied from the melt and gaining strength upon solidification (see "Adhesion and Adhesives Technology: An Introduction" by Alphonsus V. Pocius (Hanser publishers Munich, 1997).

"Auxiliary adhesive" refers to an adhesive used to attach either the substrate layer or cover layer of the absorbent core to the layer comprising the AGM particles.

An absorbent article comprising an absorbent core according to the present invention can be for example a sanitary napkin or a panty liner.

### Absorbent Articles

A typical absorbent article comprises a topsheet 30, a backsheet 40 and an absorbent core interposed between the topsheet 30 and the backsheet 40.

Typically, the topsheet 30 is oriented towards the wearer and the backsheet 40 is oriented towards the garment when the absorbent article is worn. The topsheet 30 may at least partially be in contact with the skin of the wearer when the absorbent article is worn.

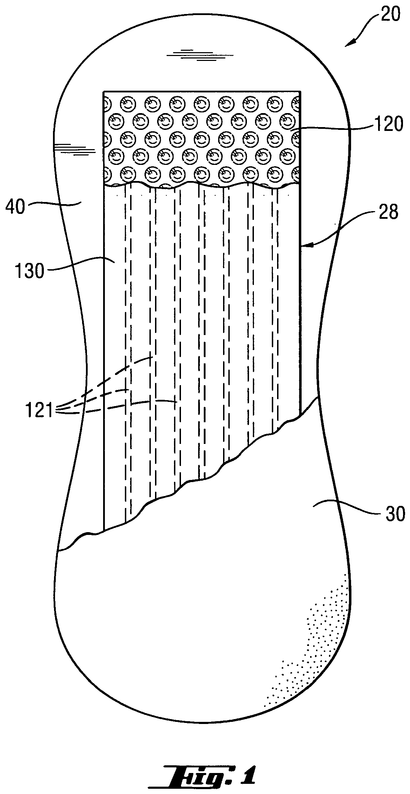

An embodiment of an absorbent article comprising the absorbent core described herein, a sanitary napkin 20, is shown in Figure 1. Typically, a sanitary napkin 20 comprises the elements of a liquid pervious topsheet 30, a backsheet 40 and an absorbent core 28 interposed between the topsheet 30 and the backsheet 40. In order to reveal the underlying structure of the absorbent article 20, the topsheet 30 is shown partially cut away to reveal the absorbent core 28. In typical embodiments, the absorbent core 28 comprises a cover layer 130, a substrate layer 100, an absorbent layer comprising a layer of superabsorbent polymer particles, or for ease of reference AGM particles 110 and a layer of a thermoplastic adhesive 120. Further, the absorbent core 28 comprises a layer of a first auxiliary adhesive 121 which is indicated as an exemplary embodiment in a pattern of stripes below the cover layer 130. In figure 1, the cover layer 130, together with the stripes of the layer of the first auxiliary adhesive 121, are shown partially cut away to reveal the underlying layer of thermoplastic adhesive 120.

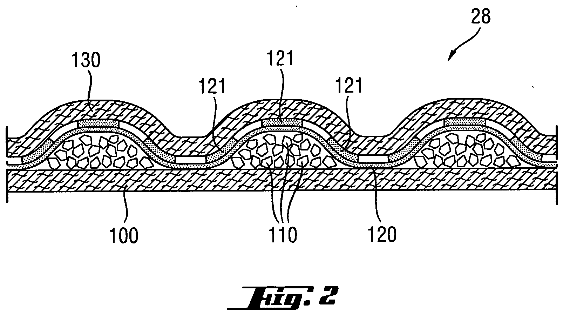

In figure 2 an embodiment of the absorbent core 28 is shown in a cross sectional view. The absorbent core 28 comprises a substrate layer 100, an absorbent layer comprising a layer of AGM particles 110 and a layer of a thermoplastic adhesive 120, a layer of a first auxiliary adhesive 121 which may be disposed for example in a pattern of stripes or dots and a cover layer 130.

### Topsheet

The absorbent article comprises a liquid pervious topsheet 30. Suitable topsheets may be woven or non-woven fabrics and/or three-dimensional webs made of a liquid impermeable polymeric film comprising liquid permeable apertures. For example, the wearer-facing and contacting surface can be provided by a film material having apertures which are provided to facilitate liquid transport from the wearer facing surface towards the absorbent structure. Such liquid permeable, apertured films provide a resilient three-dimensional fibre-like structure. Such films have been disclosed in detail for example in US 3929135, US 4151240, US 4319868, US 4324426, US 4343314, US 4591523, US 4609518, US 4629643, US 4695422 or WO 96/00548.

The topsheet may be a single layer or may comprise a multiplicity of layers.

### Absorbent Core

An absorbent core 28 generally has two sides, a first side and a second side, a length along an x-axis (longitudinal axis), a width, typically smaller than the length, along a y-axis (transversal axis) and a height, smaller than the width, along a z-axis.

The absorbent core 28 may be of rectangular shape, or alternatively the core may have curved edges. For example, the core may be of an elliptical or hourglass shape.

The absorbent core 28 is oriented such that its first side is oriented towards the wearer and its second side is oriented towards the garment of the wearer when the absorbent article is worn. In such embodiment, the first side may also be referred to as body facing side and the second side may also be referred to as garment facing side.

Surfaces of layers or materials comprised by the absorbent core 28 that are oriented toward the first side of the absorbent core 28 will be denoted first surface of the respective layer, surfaces oriented towards the second side of the absorbent core 28 will be denoted as second surface.

The absorbent core 28 comprises a cover layer 130 having a first and a second surface, a layer of a first auxiliary adhesive 121 deposited on the second surface of the cover layer 130, a substrate layer 100 having a first and a second surface, a layer of super absorbent polymer material (AGM), typically in the form of superabsorbent polymer particles (AGM particles) 110, and a layer of a thermoplastic adhesive 120. Optionally, the absorbent core 28 comprises a layer of a second auxiliary adhesive deposited on the first surface of the substrate layer 100.

The super absorbent polymer particles 110 and the thermoplastic adhesive 120 are comprised between the second surface of the cover layer 130 and the first surface of the substrate layer 100.

In order to describe typical structures of the absorbent core 28, the AGM particles 110, the thermoplastic adhesive 120, as well as the first auxiliary adhesive 121 and the optional second auxiliary adhesive are referred to herein as "layers" having first and second surfaces, respectively. However, these layers may be discontinuous, such as for example the layer of the first auxiliary adhesive 121 or the layer of AGM particles 110. With "discontinuous layer" it is meant that the layer may comprise open areas which are substantially free of the material of the layer itself. 110.

The layer of thermoplastic adhesive 120 and the layer of AGM particles 110 may be referred to collectively as absorbent layer, which in turn also comprises a first and a second surface.

The layer of thermoplastic adhesive 120 may be provided in the form of microfibers.

### Cover layer

The second surface of the cover layer 130 is at least partially in contact with the layer of the first auxiliary adhesive 121. As described in further detail below, the layer of the first auxiliary adhesive 121 is typically applied in form of a pattern. Thus, the second surface of the cover layer may also be partially in contact with the first surface of the absorbent layer.

Exemplary materials for the cover layer 130 can be provided by nonwoven materials comprising synthetic fibres, such as polyethylene (PE), polyethylene terephthalate (PET), polypropylene (PP). As the polymers used for nonwoven production are inherently hydrophobic, they can be typically coated with hydrophilic coatings, for example with durably hydrophilic coatings to provide permanently hydrophilic nonwovens. Other nonwoven materials for the optional cover layer 130 can comprise composite structures such as a so called SMS material, comprising a spunbonded, a melt-blown and a further spunbonded layer. The material of the cover layer 130 can also comprise cellulose or cellulose derivative fibres, typically from 50% to 95% by weight, or from 60% to 80% by weight, for example 70% by weight of cellulose or cellulose derivative fibres.

Basis weights for the materials of the cover layer 130 can typically range from 5 g/m² to 80 g/m², or from 10 g/m² to 60 g/m², or also from 20 g/m² to 40 g/m².

### Auxiliary adhesive

As will be described below, the thermoplastic adhesive 120 is used to immobilize the AGM particles 110. However, as AGM particles 110 tend to swell upon absorption of liquids, the thermoplastic adhesive 120 should not exert strong constraining forces to the AGM particles, but be flexible and extensible in order to accommodate the swelling of the particles.

Typically, the thermoplastic adhesive 120 may be deposited in microfibers to at least partially cover and enlace the AGM particles 110 in order to immobilize them in the dry state and in the wet state. The microfibrous structure is further advantageous as the microfibers can typically expand in response to swelling of the AGM particles 110 without breaking which improves wet immobilization.

However, for example due to their small width, such microfibers may not provide enough bond strength to adhere well to the substrate layer 100 and / or to the cover layer 130, which may lead to absorbent cores with a lower integrity than desired. At the same time, providing a greater amount of the, typically microfibrous, thermoplastic adhesive 120 in order to improve the integrity of the absorbent core, may be slightly detrimental for the fluid handling capacity of the absorbent core, especially with regard to complex fluids such as menses or blood.

To improve the integrity of the absorbent core 28 while keeping the fluid handling capacity of the core, such as acquisition and / or absorption, towards bodily fluids such as menses, it may thus be advantageous to adhere the absorbent layer, which comprises the layer of thermoplastic adhesive 120 and the layer of AGM particles 110, to the cover layer 130 and, optionally, to the substrate layer 100. This can be done by using an additional adhesive, which will herein be referred to as auxiliary adhesive. A layer of a first auxiliary adhesive 121 is applied to the cover layer 130. Optionally, a layer of a second auxiliary adhesive may be applied to the substrate laver. Thereby, the first auxiliary adhesive 121 and the optional second auxiliary adhesive may act as a bridge between the cover layer, 130 and optionally also the substrate layer, 100 and the absorbent layer.

The first and the optional second auxiliary adhesives can thus be selected and applied in order to adhere to the adjacent layers and / or materials, without the need of also being suitable to accommodate the swelling of the AGM particles 110. For example, the first auxiliary adhesive 121 does not need to be applied in the form of microfibers.

In typical embodiments of the absorbent core 28, fluids need to pass through the cover layer 130 of the absorbent core 28 first before they can be absorbed by the underlying absorbent layer. However, especially with regard to menses, which tend to be more viscous and typically have a higher surface tension when compared to urine, the application of the first auxiliary adhesive 121 may block the passage of these fluids and thus lead to increased acquisition times.

It has been found that, in order to allow for menses to more quickly pass through the cover layer 130 into the absorbent core 28, the first auxiliary adhesive 121 which is applied to the second surface of the cover layer 130 is advantageously applied in a pattern.

"Pattern" herein refers to a discontinuous layer of the first auxiliary adhesive 121 being arranged such that areas of the second surface of the cover layer 130 are covered by the first auxiliary adhesive 121 while other areas are not covered by the first auxiliary adhesive 121.

Thus, a portion of the surface area of the second surface of the cover layer 130 is covered with the first auxiliary adhesive 121.

The layer of the first auxiliary adhesive 121 may be deposited for example in a pattern of stripes or dots. The stripes may be curved or substantially straight. In some embodiments the layer of the first auxiliary adhesive 121 may be deposited in intersecting or non intersecting stripes. For example, as shown in Fig.1, the layer of the first auxiliary adhesive 121 may be deposited in a pattern of stripes running substantially parallel to the longitudinal axis of the absorbent core 28.

In order to balance the passage of fluids through the cover layer 130 with an improved integrity, the layer of the first auxiliary adhesive 121 is deposited in a pattern such that less than 35% of the surface area of the second surface of the cover layer 130 and more than 25% of the surface area of the second surface of the cover layer 130 are covered by the layer of the first auxiliary adhesive 121. For example 33 % of the surface area of the second surface of the cover layer 130 may be covered by the layer of the first auxiliary adhesive 121.

In embodiments wherein the pattern comprises stripes, the stripes may have a width of from 0.5 to 3 mm, or from 0.7 to 1.3 mm, for example 1 mm. Further, the stripes my be spaced apart from each other by 1 to 5 mm, or by 2 to 4 mm, for example by 3 mm.

The first surface of the layer of the first auxiliary adhesive 121 is at least partially in contact with the second surface of the cover layer 130 and the second surface of the layer of first auxiliary adhesive 121 may be at least partially in contact with the first surface of the absorbent layer.

Further, the second surface of the layer of the first auxiliary adhesive 121 is at least partially in contact with the first surface of the layer of the thermoplastic adhesive 120.

The first auxiliary adhesive 121 present in the absorbent core 28 may have an overall basis weight of from of from 4 g/m² to 11 g/m², or from 7 g/m² to 9 g/m², for example 8 g/m².

Optionally, the absorbent core 28 may additionally comprise a layer of a second auxiliary adhesive deposited on the first surface of the substrate layer 100. The layer of the second auxiliary adhesive may be deposited in a discontinuous layer, i.e. in any of the patterns described with regard to the first auxiliary adhesive 121, as well as in a substantially continuous layer. The layer of the second auxiliary adhesive may for example be applied by spray coating. The layer of the second auxiliary adhesive may be continuous.

In such embodiments at least a portion of the surface area of the first surface of the substrate layer 100 may be covered by the layer of the second auxiliary adhesive.

The layer of the second auxiliary adhesive may be deposited such that at least 70%, or alternatively from 80% to 95%, of the surface area of the first surface of the substrate layer 100 is covered by the layer of the second auxiliary adhesive.

Typical adhesive materials for the auxiliary adhesives may for example be construction adhesives. A typical adhesive material for the first auxiliary adhesive 121 and the optional second auxiliary adhesive can have for example a Brookfield viscosity measured according to the test method ASTM D 3236/88 at 149°C, spindle 27.5 at 30 rpm, from 2500 cP to 3800 cP, or from 2800 cP to 3200 cP, for example 2950 cP. A typical adhesive material for the first and the optional second auxiliary adhesive can also have a Ring and Ball softening point, measured according to the test method ASTME-28-99, from 80°C to 100°C, or also from 86°C to 91°C. A suitable auxiliary adhesive is for example Bostik H4265, supplied by Bostik Netherlands.

Typically, the Ring and Ball softening point of the first and the optional second auxiliary adhesive can be lower than the Ring and Ball softening point of the thermoplastic adhesive, measured at the same conditions.

In one simplified embodiment, the adhesive material described for the first and the optional second auxiliary adhesive may also be used for the layer of thermoplastic adhesive 120. However, also in such an embodiment the layer of thermoplastic adhesive 120 is typically provided in microfibers.

### Absorbent layer

The absorbent layer comprises the layer of super absorbent polymer particles (AGM particles) 110 and the layer of thermoplastic adhesive 120. The layer of AGM particles 110 may be a uniform or non uniform layer, wherein by "uniform" or "non uniform" it is meant that the AGM particles 110 can be distributed over the substrate layer 100 with uniform or non uniform basis weight.

According to the present invention, the layer of AGM particles 110 may be a discontinuous layer. With "discontinuous layer" it is meant that the layer comprises open areas which are substantially free of super absorbent polymer material. For example, the layer of AGM particles 110 may comprise openings, i.e. the open areas which may be surrounded by areas comprising AGM particles 110. Typically these openings have a diameter or largest span of less than 10 mm, or less than 5 mm, or 3 mm, or 2 mm, or 1.5 mm and of more than 0.5 mm, or 1 mm. Alternatively, the absorbent layer may comprise areas comprising AGM particles 110 which are surrounded by open areas. In certain embodiments, the areas comprising AGM particles 110 may be discrete areas having a diameter or largest span of less than 10 mm, or less than 5 mm, or 3 mm, or 2 mm, or 1.5 mm and of more than 0.5 mm, or 1 mm.

When the layer of AGM particles 110 is provided as a non uniform layer, typically for example as a discontinuous layer, some portions of the first surface of the substrate layer 100 may not be covered by AGM particles 110.

The layer of the thermoplastic adhesive 120 serves to at least partially immobilize the AGM particles 110. Typically, the layer of the thermoplastic adhesive 120 may be provided in the form of fibres, such as microfibers.

In embodiments comprising a discontinuous layer of AGM particles 110, the layer of the thermoplastic adhesive 120 may be at least partially in contact with the layer of AGM particles 110 and also at least partially in contact with the substrate layer 100.

For example, when a layer of thermoplastic adhesive 120 is laid down onto the first surface of the layer of AGM particles 110, the second surface of the thermoplastic adhesive layer is at least partially in contact with the first surface of the layer of AGM particles 110, but also at least partially in contact with the first surface of the substrate layer 100, as shown in Fig. 2. Stated differently, the second surface of the layer of thermoplastic adhesive 120 may be in contact with the first surface of the substrate layer 100 in portions corresponding to the openings of the discontinuous layer of the AGM particles 110. Typically, the layer of the thermoplastic adhesive 120 may be in contact with the substrate layer 100 through the optional layer of the second auxiliary adhesive.

Typically, the layer of thermoplastic adhesive 120 may undulate between the first surface of the layer of AGM particles 110 and the first surface of the substrate layer 100. The areas where the layer of thermoplastic adhesive 120 is in contact with the substrate layer 100 are referred to as the areas of junction.

Exemplary typical thermoplastic adhesives 120 and typical superabsorbent polymer particles 110 are further described in their respective sections below.

### Substrate layer

In typical embodiments, the substrate layer 100 may be at least partially in contact with the layer of AGM particles 110. For example, the second surface of the layer of AGM particles 110 may be at least partially in contact with the first surface of the substrate layer 100.

Exemplary materials for the substrate layer 100 may comprise nonwoven materials comprising synthetic fibres, or natural fibres, or mixtures thereof, such as for example carded nonwovens, or more typically airlaid or wetlaid fibrous materials, such as for example latex or thermal bonded airlaid fibrous materials, comprising synthetic and natural fibres, such as for example cellulose fibres.

For example, the substrate layer 100 may comprise a fibrous material comprising cellulose or cellulose derivative fibres, typically for example from about 40% to about 100% by weight of cellulose or cellulose derivative fibres, or from about 50% to about 95% by weight of cellulose or cellulose derivative fibres, or also from about 60% to about 90% by weight of cellulose or cellulose derivative fibres.

A substrate layer 100 comprising a substantial percentage of cellulose fibres can provide an advantage in terms of liquid distribution to the fraction of liquid which is not immediately absorbed by the absorbent layer.

Basis weights for the materials of the substrate layer 100 can typically range from about 10 g/m² to about 120 g/m², or from about 40 g/m² to about 100 g/m², or also from about 50 g/m² to about 80 g/m².

### Thermoplastic adhesive

The thermoplastic adhesive 120 present in the absorbent layer may have a basis weight of from 11 g/m² to 3 g/m², preferably of from 9 g/m² to 5 g/m², for example 8 g/m², or 6 g/m².

Typically, the layer of the thermoplastic adhesive 120 can be applied in form of microfibers to enlace the absorbent material, such as the super absorbent polymer particles 110. For example, the microfibers may have an average thickness of from 1 µm to 100 µm, or from 25 µm to 75 µm.

Without wishing to be bound by theory it has been found that hot melt adhesive materials which show good cohesion behaviour may be used as thermoplastic adhesive 120. Good cohesion ensures that the adhesive does not break, in particular in response to external forces, and namely in response to strain. The thermoplastic adhesive 120 is subject to external forces when the absorbent product has acquired liquid, which is then stored in the AGM particles 110 which in response swell. An exemplary thermoplastic adhesive 120 should allow for such swelling, without breaking and without imparting too many compressive forces, which would restrain the AGM particles 110 from swelling. It may be desirable that the thermoplastic adhesive 120 does not break, which would deteriorate the wet immobilization of the AGM particles 110. Exemplary suitable hot melt adhesive materials can be as described in the already mentioned patent application EP 1447067, particularly at sections [0052] to [0063].

In particular, a typical adhesive material for the thermoplastic adhesive can meet one, or several, or also all of the parameters in respective sections [0058] to [0061] of the above mentioned patent application EP 1447067. In addition or alternatively, the thermoplastic adhesive can meet one or several or all of the parameters described below, i.e. the cohesive strength parameter γ, the Brookfield viscosity and the Ring and Ball softening point.

For example, the thermoplastic adhesive 120 may have a sufficient cohesive strength parameter γ. The cohesive strength parameter γ is measured using the Rheological Creep Test as referred to hereinafter. A sufficiently low cohesive strength parameter γ is representative of elastic adhesive which, for example, can be stretched without tearing. If a stress of τ = 1000 Pa is applied, the cohesive strength parameter γ can be less than 100%, less than 90%, or less than 75%. For a stress of τ = 125000 Pa, the cohesive strength parameter γ can be less than 1200%, less than 1000%, or less than 800%.

A typical adhesive material for the thermoplastic adhesive can also have a Brookfield viscosity measured according to the test method ASTM 12-3236/88 at 149°C, spindle 27.5 at 30 rpm, from 2100 cP to 2800 cP, for example 2500 cP.

For example, the Brookfield viscosity of the thermoplastic adhesive 120 can be lower than the Brookfield viscosity of the first auxiliary adhesive 121 and the optional second auxiliary adhesive.

A typical adhesive material for the thermoplastic adhesive can also have a Ring and Ball softening point, measured according to the test method ASTM E-28-99, from 105°C to 120°C, or also from 107°C to 115°C.

### Super absorbent polymer particles (AGM particles)

Typically, the layer of AGM particles can be constituted by 100% by weight super absorbent polymer particles 110.

Alternatively, the AGM particles 110 may also be optionally mixed with fibrous material, which can provide a matrix for further immobilization of the AGM particles. However, typically a relatively low amount of fibrous material can be used, for example less than about 40 weight %, less than about 20 weight %, or less than about 10 weight % of the total weight of the AGM particles.

AGM particles 110 comprised in the absorbent layer typically comprise substantially water-insoluble, water-swellable polymer particles, which may also be porous.

According to an embodiment, porous AGM particles 110 can be selected among polyacrylates and polyacrylate based materials, such as for example partially neutralized, crosslinked polyacrylates.

In one embodiment the layer of AGM particles 110 in the absorbent core 28 is present throughout the area of the absorbent core 28 in an average basis weight of less than about 250 g/m², or of less than about 220 g/m², or from about 60 g/m² to about 180 g/m², or from about 100 g/m² to about 160 g/m². Average basis weights for the layer of AGM particles 110 of up to about 300 g/m², or up to about 400 g/m², or also up to about 500 g/m² can also be used. An average basis weight is typically based on the whole area of the zone of application, i.e. interested by the layer of AGM particles 110, and hence comprising possible openings included in an e.g. discontinuous layer. Typically, the AGM particles can constitute at least about 45%, or at least about 50%, or at least about 55%, by weight of the absorbent core 28.

The AGM particles 110 can typically have a selected average particle size from about 200 µm to about 600 µm, or from about 300 µm to about 500 µm.

The average particle size of the AGM particles can be determined as it is known in the art, for example by means of dry sieve analysis. Optical methods, e.g. based on light scattering and image analysis techniques, can also be used.

According to an embodiment, the AGM particles 110 can be selected among the polyacrylate based polymers described in the PCT Patent Application WO 07/047598, which are polyacrylate based materials very slightly crosslinked, or substantially not crosslinked at all. Particularly, said polyacrylate based materials can have an extractable fraction of at least about 30% by weight, between about 30% and about 80% by weight, or between about 32% and about 70% by weight, evaluated according to the Extractables test method described in the above referenced application. Alternatively, said polyacrylate based materials can have a retention capacity of at least about 30 g/g, at least about 35 g/g, or at least about 40 g/g, evaluated according to the Centrifuge Retention Capacity test described in the above referenced application. The AGM particles 110 can also be selected among the polyacrylate based polymers described in the PCT Patent Application WO 07/046052. Said polymers in fact are particularly effective in absorbing complex body fluids such as menses or blood, and upon absorption of such fluids do not generally show a marked swelling, followed by gel blocking, like traditional super absorbents, but rather act to a certain extent as thickeners of the body fluid, immobilizing it as a sort of gelatinous mass within the absorbent structure, for example in the interstices among the fibres, without causing substantial swelling and in turn a sensible increase of the overall thickness of the absorbent core 28. Said polymers can be provided in form of porous absorbent polymer particles according to known methods.

The AGM particles for the absorbent cores may have a permeability, as expressed by the saline flow conductivity of the AGM particles, greater than 10, 20, 30 or 40 SFC- units, where 1 SFC unit is 1 × 10⁻⁷ (cm³ × s) / g. Saline flow conductivity is a parameter well recognised in the art and is to be measured in accordance with the test disclosed in EP 752 892 B.

In certain embodiments, the absorbent core 28 described above can fully constitute the absorbent element in an absorbent article, or can be complemented with additional layers. For example a fibrous acquisition layer may be deposited between the absorbent core 28 and the topsheet 30. According to an embodiment, the acquisition layer can for example comprise fibrous nonwoven materials made by air laying or wet laying of synthetic fibres such as polyethylene (PE), polyethylene terephthalate (PET), or polypropylene (PP), similarly to the cover layer 130 of the absorbent core 28 of the present invention.

Exemplary materials for the fluid acquisition layer could comprise spunbonded or carded nonwoven materials, or airlaid materials such as for example latex bonded or thermal bonded airlaid materials. Basis weights can typically range from about 10 g/m² to about 60 g/m², or from about 25 g/m² to about 40 g/m².

According to another alternative embodiment the absorbent article can comprise a further fibrous layer comprised between the absorbent core 28 and the backsheet 40, i.e. typically provided at the garment facing surface of the core. This optional layer can be provided by similar fibrous materials as those already described for the substrate layer 100 of the absorbent core 28 of the present invention. The optional fibrous layer according to this further embodiment can act as an added wicking layer receiving and distributing excess fluid which might not be fully retained by the absorbent core 28.

Further materials, also typically in particle form, can be comprised in the absorbent core 28 for example known odour control materials, or inert materials such as silica.

### Backsheet

The absorbent article may also comprise a backsheet 40. The backsheet 40 may be used to prevent the fluids absorbed and contained in the absorbent structure from wetting materials that contact the absorbent article such as underpants, pants, pyjamas, undergarments, and shirts or jackets, thereby acting as a barrier to fluid transport. The backsheet 40 can also allow the transfer of at least water vapour, or both water vapour and air through it.

Especially when the absorbent article finds utility as a sanitary napkin or panty liner, the absorbent article can be also provided with a panty fastening means, which provides means to attach the article to an undergarment, for example a panty fastening adhesive on the garment facing surface of the backsheet. Wings or side flaps meant to fold around the crotch edge of an undergarment can be also provided on the side edges of the napkin.

### Process for making an absorbent core

A process for making the absorbent core 28 described herein will now be described.

### Step A)

A cover layer having a first and a second surface is provided and a layer of a first auxiliary adhesive is deposited on the second surface of the cover layer. The layer of the first auxiliary adhesive is deposited in a pattern such that less than 35% and more than 25%, such as 33 % of the surface area of the second surface of the cover layer is covered by the layer of the first auxiliary adhesive.

In certain embodiments, the layer of the first auxiliary adhesive is deposited in a pattern of stripes having a width of from 0.5 to 3 mm, or from 0.7 to 1.3 mm, for example 1 mm. Further, the stripes my be spaced apart from each other by 1 to 5 mm, or by 2 to 4 mm, for example by 3 mm.

### Step B)

Providing a substrate layer having a first and a second surface.

The substrate layer may for example provided form a continuous source of material, such as a roll.

### Step C) (optional)

Optionally, a layer of a second auxiliary adhesive is provided and deposited on the first surface of the substrate layer. For example, the layer of the second auxiliary adhesive may be deposited onto the first surface of the substrate layer by spray-coating.

### Step D)

Providing a layer of superabsorbent polymer particles and depositing it onto the first surface of the substrate layer. As already pointed out, the first surface of the substrate layer may optionally have been pre-treated with the second auxiliary adhesive.

### Step E)

Depositing a layer of thermoplastic adhesive onto the layer of super absorbent polymer particles and the substrate layer to cover the onto the layer of super absorbent polymer particles on the first surface of the substrate layer;

### Step F)

Assembling the absorbent core such that the layer of first auxiliary adhesive is sandwiched between the second surface of the cover layer and the layer of the thermoplastic adhesive.

### Test Method

### Rheological Creep Test

The Rheological Creep Test mentioned hereinabove for measuring the cohesive strength parameter γ is as described in EP 1447067, assigned to the Procter & Gamble Company.

## Claims

1. An absorbent feminine hygiene product comprising a first surface oriented toward the body of the wearer and a second surface oriented toward the garment of the wearer
said absorbent product comprising a topsheet, a backsheet and an absorbent core interposed in between, said absorbent core (28) having a longitudinal and a transversal axis, the absorbent core (28) being formed by the following layers:
a cover layer (130) having a first and a second surface,
a layer of first auxiliary adhesive (121) deposited on the second surface of the cover layer (130),
a substrate layer (100) having a first and a second surface,
an absorbent layer comprising a layer of super absorbent polymer particles (110) and a layer of thermoplastic adhesive (120);
an optional layer of second auxiliary adhesive deposited on the first surface of the substrate layer (100)
wherein the layer of super absorbent polymer particles (110) is comprised between the second surface of the cover layer (130) and the first surface of the substrate layer (100);
wherein the layer of thermoplastic adhesive (120) covers the layer of super absorbent polymer particles (110) on the first surface of the substrate layer (100);
and wherein the first auxiliary adhesive (121) is deposited in a pattern to cover less than 35% and more than 25% of the surface area of the second surface of the cover layer (130).

2. The absorbent feminine hygiene product according to claim 1, wherein in the core (28) the layer of the first auxiliary adhesive (121) is deposited in a pattern of stripes having a width of from 0.5 to 3 mm, preferably from 0.7 to 1.3 mm.

3. The absorbent feminine hygiene product according to claim 1 or 2, wherein in the core (28) the layer of the first auxiliary adhesive (121) is deposited in a pattern of stripes and wherein the stripes are parallel.

4. The absorbent feminine hygiene product according to claim 3, wherein the stripes are spaced apart from each other by 1 to 5 mm, preferably by 2 to 4 mm.

5. The absorbent feminine hygiene product of claim 3 or 4, wherein the stripes are parallel to the longitudinal axis of the absorbent core (28).

6. The absorbent feminine hygiene product according to any of the preceding claims, wherein in the core (28) the layer of thermoplastic adhesive (120) is provided in the form of micro-fibers having an average thickness of from 1 µm to 100 µm, preferably from 25 µm to 75 µm.

7. The absorbent feminine hygiene product according to any of the preceding claims, wherein the core (28) further comprises a layer of second auxiliary adhesive deposited on the first surface of the substrate layer (100).

8. The absorbent feminine hygiene product according to claim 7, wherein the first and second auxiliary adhesive comprise the same adhesive material.

9. The absorbent feminine hygiene product according to any of the preceding claims, wherein in the core (28) the cover layer (130) is a nonwoven web.

10. The absorbent feminine hygiene product according to any of the preceding claims, wherein in the core (28) the substrate layer (100) is a carded, airlaid or wetlaid nonwoven web comprising synthetic fibres, or natural fibres, or mixtures of synthetic and natural fibers.

11. The absorbent feminine hygiene product according to any of the preceding claims, wherein in the core (28) the cover layer (130) comprises from 50% to 95% by weight, or from 60% to 80% by weight, for example 70% by weight of cellulose or cellulose derivative fibers.

12. The absorbent feminine hygiene product according to any of the preceding claims, wherein in the core (28) the super absorbent polymer particles (110) are selected from partially neutralized, crosslinked polyacrylates.

13. The absorbent feminine hygiene product according to any of the preceding claims, wherein the core (28) comprises less than 40 weight %, preferably less than 20 weight %, more preferably less than 10 weight % of the total weight of the super absorbent polymer particles (110) of fibrous material.

14. The absorbent feminine hygiene product according to any preceding claim, wherein the absorbent feminine hygiene product is a sanitary napkin (20).

15. A process for making the absorbent core for the absorbent feminine hygiene product of any preceding claim comprising the steps of
A) providing a cover layer having a first and a second surface and depositing a layer of first auxiliary adhesive on the second surface of the cover layer;
wherein the layer of the first auxiliary adhesive is deposited in a pattern to cover less than 35% and more than 25%;
B) providing a substrate layer having a first and a second surface;
C) optionally providing a layer of a second auxiliary adhesive on the first surface of the substrate layer;
D) providing a layer of super absorbent polymer particles onto the first surface of the substrate layer and the optional layer of second auxiliary adhesive;
E) depositing a layer of a thermoplastic adhesive onto the layer of super absorbent polymer particles and the substrate layer to cover the layer of super absorbent polymer particles on the first surface of the substrate layer;
F) assembling the absorbent core such that the layer of first auxiliary adhesive is sandwiched between the second surface of the cover layer and the layer of the thermoplastic adhesive.

## Patentansprüche

1. Absorbierendes Damenhygieneprodukt, umfassend eine erste Oberfläche, die zum Körper des Trägers ausgerichtet ist, und eine zweite Oberfläche, die zum Kleidungsstück des Trägers ausgerichtet ist
wobei das Absorptionsprodukt eine Oberschicht, eine Unterschicht und einen dazwischen eingeschobenen Absorptionskern umfasst, wobei der Absorptionskern (28) eine Längs- und eine Querachse aufweist, wobei der Absorptionskern (28) durch die folgenden Schichten gebildet wird:
eine Deckschicht (130), die eine erste und eine zweite Oberfläche aufweist,
eine Schicht aus erstem Hilfsklebstoff (121), die an der zweiten Oberfläche der Deckschicht (130) angelagert ist,
eine Substratschicht (100), die eine erste und eine zweite Oberfläche aufweist,
eine Absorptionsschicht, umfassend eine Schicht aus Superabsorptionspolymerteilchen (110) und eine Schicht aus Thermoplast-Klebstoff (120);
eine wahlweise Schicht aus zweitem Hilfsklebstoff, der an der ersten Oberfläche der Substratschicht (100) angelagert ist
wobei die Schicht aus Superabsorptionspolymerteilchen (110) zwischen der zweiten Oberfläche der Deckschicht (130) und der ersten Oberfläche der Substratschicht (100) umfasst ist;
wobei die Schicht aus Thermoplast-Klebstoff (120) die Schicht aus Superabsorptionspolymerteilchen (110) auf der ersten Oberfläche der Substratschicht (100) bedeckt;
und wobei der erste Hilfsklebstoff (121) in einem Muster angelagert ist, um weniger als 35 % und mehr als 25 % des Oberflächenbereichs der zweiten Oberfläche der Deckschicht (130) zu bedecken.

2. Absorbierendes Damenhygieneprodukt nach Anspruch 1, wobei in dem Kern (28) die Schicht des ersten Hilfsklebstoffes (121) in einem Streifenmuster, das eine Breite von 0,5 bis 3 mm, vorzugsweise von 0,7 bis 1,3 mm aufweist, angelagert ist.

3. Absorbierendes Damenhygieneprodukt nach Anspruch 1 oder 2, wobei in dem Kern (28) die Schicht des ersten Hilfsklebstoffes (121) in einem Streifenmuster angelagert ist, und wobei die Streifen parallel verlaufen.

4. Absorbierendes Damenhygieneprodukt nach Anspruch 3, wobei die Streifen um 1 bis 5 mm, vorzugsweise um 2 bis 4 mm voneinander beabstandet sind.

5. Absorbierendes Damenhygieneprodukt nach Anspruch 3 oder 4, wobei die Streifen parallel zu der Längsachse des Absorptionskerns (28) verlaufen.

6. Absorbierendes Damenhygieneprodukt nach einem der vorstehenden Ansprüche, wobei in dem Kern (28) die Schicht aus Thermoplast-Klebstoff (120) in Form von Mikrofasern, die eine durchschnittliche Dicke von 1 µm bis 100 µm, vorzugsweise von 25 µm bis 75 µm aufweisen, bereitgestellt ist.

7. Absorbierendes Damenhygieneprodukt nach einem der vorstehenden Ansprüche, wobei der Kern (28) ferner eine Schicht aus zweitem Hilfsklebstoff umfasst, der an der ersten Oberfläche der Substratschicht (100) angelagert ist.

8. Absorbierendes Damenhygieneprodukt nach Anspruch 7, wobei der erste und der zweite Hilfsklebstoff das gleiche Klebstoffmaterial umfassen.

9. Absorbierendes Damenhygieneprodukt nach einem der vorstehenden Ansprüche, wobei in dem Kern (28) die Deckschicht (130) eine Vliesbahn ist.

10. Absorbierendes Damenhygieneprodukt nach einem der vorstehenden Ansprüche, wobei in dem Kern (28) die Substratschicht (100) eine kardierte, luftgelegte oder nassgelegte Vliesbahn ist, umfassend Synthetikfasern oder Naturfasern oder Mischungen aus Synthetik- und Naturfasern.

11. Absorbierendes Damenhygieneprodukt nach einem der vorstehenden Ansprüche, wobei in dem Kern (28) die Deckschicht (130) zu 50 Gew.-% bis 95 Gew.-% oder zu 60 Gew.-% bis 80 Gew.-%, zum Beispiel zu 70 Gew.-%, Cellulose oder Cellulosederivatfasern, umfasst.

12. Absorbierendes Damenhygieneprodukt nach einem der vorstehenden Ansprüche, wobei in dem Kern (28) die Superabsorptionspolymerteilchen (110) ausgewählt sind aus teilweise neutralisierten vernetzten Polyacrylaten.

13. Absorbierendes Damenhygieneprodukt nach einem der vorstehenden Ansprüche, wobei der Kern (28) zu weniger als 40 Gew.-%, vorzugsweise zu weniger als 20 Gew.-%, mehr bevorzugt zu weniger als 10 Gew.-% des Gesamtgewichts der Superabsorptionspolymerteilchen (110) Fasermaterial umfasst.

14. Absorbierendes Damenhygieneprodukt nach einem der vorstehenden Ansprüche, wobei das absorbierende Damenhygieneprodukt eine Damenbinde (20) ist.

15. Verfahren zur Herstellung des Absorptionskerns für das absorbierende Damenhygieneprodukt nach einem der vorstehenden Ansprüche, umfassend die Schritte
A) Bereitstellen einer Deckschicht, die eine erste und eine zweite Oberfläche aufweist, und Anlagern einer Schicht aus erstem Hilfsklebstoff an der zweiten Oberfläche der Deckschicht;
wobei die Schicht des ersten Hilfsklebstoffes in einem Muster angelagert wird, um weniger als 35 % und mehr als 25 % zu bedecken;
B) Bereitstellen einer Substratschicht, die eine erste und eine zweite Oberfläche aufweist;
C) wahlweise Bereitstellen einer Schicht eines zweiten Hilfsklebstoffes auf der ersten Oberfläche der Substratschicht;
D) Bereitstellen einer Schicht aus Superabsorptionspolymerteilchen auf der ersten Oberfläche der Substratschicht und der wahlweisen Schicht von zweitem Hilfsklebstoff;
E) Anlagern einer Schicht eines Thermoplast-Klebstoffes an der Schicht von Superabsorptionspolymerteilchen und der Substratschicht, um die Schicht aus Superabsorptionspolymerteilchen auf der ersten Oberfläche der Substratschicht zu bedecken;
F) Zusammenfügen des Absorptionskerns auf derartige Weise, dass die Schicht aus erstem Hilfsklebstoff zwischen der zweiten Oberfläche der Deckschicht und der Schicht des Thermoplast-Klebstoffes in Sandwichform angeordnet ist.

## Revendications

1. Produit absorbant d'hygiène féminine comprenant une première surface orientée vers le corps du porteur et une deuxième surface orientée vers le vêtement du porteur
ledit produit absorbant comprenant une feuille de dessus, une feuille de fond et une âme absorbante intercalée entre elles, ladite âme absorbante (28) ayant un axe longitudinal et un transversal, l'âme absorbante (28) étant formée par les couches suivantes :
une couche de couverture (130) ayant une première et une deuxième surface,
une couche de premier adhésif auxiliaire (121) déposée sur la deuxième surface de la couche de couverture (130),
une couche de substrat (100) ayant une première et une deuxième surface,
une couche absorbante comprenant une couche de particules de polymère superabsorbant (110) et une couche d'adhésif thermoplastique (120) ;
une couche facultative de deuxième adhésif auxiliaire déposée sur la première surface de la couche de substrat (100)
dans lequel la couche de particules de polymère superabsorbant (110) est comprise entre la deuxième surface de la couche de couverture (130) et la première surface de la couche de substrat (100) ;
dans lequel la couche d'adhésif thermoplastique (120) couvre la couche de particules de polymère superabsorbant (110) sur la première surface de la couche de substrat (100) ;
et dans lequel le premier adhésif auxiliaire (121) est déposé en un motif pour couvrir moins de 35 % et plus de 25 % de la superficie de la deuxième surface de la couche de couverture (130).

2. Produit absorbant d'hygiène féminine selon la revendication 1, dans lequel dans l'âme (28) la couche du premier adhésif auxiliaire (121) est déposée en un motif de bandes ayant une largeur allant de 0,5 à 3 mm, de préférence de 0,7 à 1,3 mm.

3. Produit absorbant d'hygiène féminine selon la revendication 1 ou 2, dans lequel dans l'âme (28) la couche du premier adhésif auxiliaire (121) est déposée en un motif de bandes et dans lequel les bandes sont parallèles.

4. Produit absorbant d'hygiène féminine selon la revendication 3, dans lequel les bandes sont espacées les unes des autres de 1 à 5 mm, de préférence de 2 à 4 mm.

5. Produit absorbant d'hygiène féminine selon la revendication 3 ou 4, dans lequel les bandes sont parallèles à l'axe longitudinal de l'âme absorbante (28).

6. Produit absorbant d'hygiène féminine selon l'une quelconque des revendications précédentes, dans lequel dans l'âme (28) la couche d'adhésif thermoplastique (120) est fournie sous la forme de microfibres ayant une épaisseur moyenne allant de 1 µm à 100 µm, de préférence de 25 µm à 75 µm.

7. Produit absorbant d'hygiène féminine selon l'une quelconque des revendications précédentes, dans lequel l'âme (28) comprend en outre une couche de deuxième adhésif auxiliaire déposée sur la première surface de la couche de substrat (100).

8. Produit absorbant d'hygiène féminine selon la revendication 7, dans lequel le premier et le deuxième adhésif auxiliaire comprennent le même matériau adhésif.

9. Produit absorbant d'hygiène féminine selon l'une quelconque des revendications précédentes, dans lequel dans l'âme (28) la couche de couverture (130) est une nappe non tissée.

10. Produit absorbant d'hygiène féminine selon l'une quelconque des revendications précédentes, dans lequel dans l'âme (28) la couche de substrat (100) est une nappe non tissée cardée, appliquée par jet d'air ou appliquée par voie humide comprenant des fibres synthétiques, ou des fibres naturelles, ou des mélanges de fibres synthétiques et naturelles.

11. Produit absorbant d'hygiène féminine selon l'une quelconque des revendications précédentes, dans lequel dans l'âme (28) la couche de couverture (130) comprend de 50 % à 95 % en poids, ou de 60 % à 80 % en poids, par exemple 70 % en poids de fibres de cellulose ou dérivé de cellulose.

12. Produit absorbant d'hygiène féminine selon l'une quelconque des revendications précédentes, dans lequel dans l'âme (28) les particules de polymère superabsorbant (110) sont choisies parmi des polyacrylates réticulés, partiellement neutralisés.

13. Produit absorbant d'hygiène féminine selon l'une quelconque des revendications précédentes, dans lequel l'âme (28) comprend moins de 40 % en poids, de préférence moins de 20 % en poids, plus préférablement moins de 10 % en poids du poids total des particules de polymère superabsorbant (110) de matériau fibreux.

14. Produit absorbant d'hygiène féminine selon une quelconque revendication précédente, dans lequel le produit absorbant d'hygiène féminine est une serviette hygiénique (20).

15. Procédé de fabrication de l'âme absorbante pour le produit absorbant d'hygiène féminine selon une quelconque revendication précédente comprenant les étapes consistant à
A) fournir une couche de couverture ayant une première et une deuxième surface et déposer une couche de premier adhésif auxiliaire sur la deuxième surface de la couche de couverture ;
dans lequel la couche du premier adhésif auxiliaire est déposée en un motif pour couvrir moins de 35 % et plus de 25 % ;
B) fournir une couche de substrat ayant une première et une deuxième surface ;
C) fournir facultativement une couche d'un deuxième adhésif auxiliaire sur la première surface de la couche de substrat ;
D) fournir une couche de particules de polymère superabsorbant sur la première surface de la couche de substrat et la couche facultative de deuxième adhésif auxiliaire ;
E) déposer une couche d'un adhésif thermoplastique sur la couche de particules de polymère superabsorbant et la couche de substrat pour couvrir la couche de particules de polymère superabsorbant sur la première surface de la couche de substrat ;
F) assembler l'âme absorbante de telle sorte que la couche de premier adhésif auxiliaire est prise en sandwich entre la deuxième surface de la couche de couverture et la couche de l'adhésif thermoplastique.
